(19) Europäisches Patentamt
European Patent Office
Office européen des brevets

(11) Veröffentlichungsnummer: **0 230 575**
**A2**

(12) # EUROPÄISCHE PATENTANMELDUNG

(21) Anmeldenummer: **86116860.7**

(22) Anmeldetag: **04.12.86**

(51) Int. Cl.³: **A 61 K 31/685**
A 61 K 31/08

(30) Priorität: **04.12.85 DE 3542893**
**28.02.86 DE 3606631**

(43) Veröffentlichungstag der Anmeldung:
**05.08.87 Patentblatt 87/32**

(84) Benannte Vertragsstaaten:
**ES GR**

(71) Anmelder: **Max-Planck-Gesellschaft zur Förderung der Wissenschaften e.V.**
**Bunsenstrasse 10**
**D-3400 Göttingen(DE)**

(72) Erfinder: **Eibl, Hansjörg, Dr.**
**Steinweg 51**
**D-3406 Bovenden(DE)**

(74) Vertreter: **Huber, Bernhard, Dipl.-Chem. et al,**
**Patentanwälte H. Weickmann, Dr. K. Fincke F.A. Weickmann, B. Huber Dr. H. Liska, Dr. J. Prechtel**
**Möhlstrasse 22 Postfach 860 820**
**D-8000 München 86(DE)**

(54) **Arzneimittel mit Antitumorwirkung das Hexadecylphosphocholin enthält.**

(57) Cytotoxisch wirksame Arzneimittel, enthaltend als Wirkstoff Hexadecylphosphocholin.

EP 0 230 575 A2

Beschreibung:

BEZEICHNUNG GEÄNDERT
siehe Titelseite

Hexadecylphosphocholin ist eine bekannte Substanz. In Pharmazie 37, 1982, Seite 706-707 wird hierfür eine lysierende und fusogene Wirkung angegeben. Es wurde nun gefunden, daß diese Verbindung außerdem eine ausgezeichnete Antitumorwirkung besitzt und gegenüber homologen Verbindungen, die in der Europa-Anmeldung 108 565 beschrieben sind, sich durch folgende überraschende Eigenschaften auszeichnet: Während ähnliche Verbindungen mit einem kürzeren Alkylrest wie zum Beispiel das Tetradecylphosphocholin praktisch keine Antitumorwirkung zeigen (zum Beispiel in vitro im L 1210-Kolonien-Versuch oder in vivo am autochthanen Methylnitrosoharnstoff-induzierten Mammakarzinom der Ratte) sind solche mit einem längeren Alkylrest, wie zum Beispiel das Octadecylphosphocholin zwar antitumorwirksam, jedoch gleichzeitig viel zu toxisch und daher als Arzneimittel nicht verwendbar. So ergab sich zum Beispiel bei einer Bestimmung der subakuten Toxizität während einer Behandlung von 5 Wochen bei einer antitumorwirksamen täglichen Dosis von 77 µmol.kg Ratte oral eine extrem hohe Mortalität, die bei 80 % der gesamten Tiere lag. Trotz einer Antitumorwirkung war daher die mediane Überlebenszeit gegenüber der Kontrolle bei Octadecylphosphocholin-behandelten Tieren um 72 % verkürzt. Demgegenüber war bei Hexadecylphosphocholinbehandelten Tieren die Mortalität um die Hälfte geringer, und es kam infolge des Fehlens einer chronischen Toxizität zu einem hochsignifikanten Anstieg der medianen Überlebenszeit von 26 % gegenüber der Kontrolle.

Das Hexadecylphosphocholin nimmt also innerhalb der homologen Alkylverbindungen eine überraschende Sonderstellung ein, indem nur das Hexadecylphosphocholin eine praktisch verwertbare gute Antitumorwirkung besitzt. Homologe mit kürzeren Alkylresten besitzen keine oder eine viel zu geringe Antitumorwirkung. Homologe mit längeren Alkylresten sind zwar wirksam gegen Tumore, jedoch gleichzeitig viel zu toxisch. Allein das Hexadecylphosphocholin weist daher eine ausreichende Antitumorwirkung in nichttoxischen Dosen auf.

Die Erfindung betrifft Arzneimittel, die als Wirkstoff das Hexadecylphosphocholin enthalten und sich besonders zur Behandlung von Tumoren eignen.
Solche Arzneimittel besitzen eine ausgeprägte cytotoxische Wirksamkeit, die sowohl in vivo am chemisch induzierten Mammakarzinom der Ratte, als auch in vitro an Leukämiezellen in der Zellkultur nachgewiesen wurde. Darüberhinaus wurden in einer klinischen Pilotstudie bei Patientinnen mit Mammakarzinomen Hautmetastasen zur vollständigen Abheilung bei topischer Anwendung gebracht.

Es ist bekannt, daß bisher kein in jeder Hinsicht zufriedenstellendes Arzneimittel zur Behandlung von Tumoren, insbesondere von bösartigen Tumoren, zur Verfügung steht. So ist beispielsweise zur topischen Behandlung von Hautmetastasen bei Patienten mit metastasierenden Tumoren derzeit lediglich 5-Fluorouracil verfügbar. Weiterentwicklungen anderer Cytostatika sind für diese Applikationsart bisher nicht bis zur Klinikreife verfolgt worden. Andererseits ist aus klinischer Sicht ein solches Konzept im palliativen Therapieansatz besonders erwünscht, da alternative Behandlungskonzepte wie chirurgische Maßnahmen, Strahlentherapie und systemische Chemotherapie, vergleichsweise aggressive Therapiemodalitäten darstellen. Außerdem liegt eine beträchtliche Zahl von Patienten als potentielle Behandlungskandidaten für eine solche topische Behandlung vor. So beträgt z. B. der Anteil der Mamma-Karzinom-Patienten, die einen Hautbefall aufweisen, etwa 25 bis 35 %.

Die Voraussetzung zur topischen Behandlung seitens des einzusetzenden Wirkstoffes sind Verträglichkeit auf der Haut, cytotoxische Wirksamkeit gegen Tumorzellen und ausreichende Tiefenpenetration.

Ziel der Erfindung ist daher in erster Linie, ein Arzneimittel zu schaffen, welches zur topischen Behandlung von Tumoren geeignet ist. Ein weiteres Ziel der Erfindung ist es darüberhinaus, ein generell auch in anderen

Applikationsformen anwendbares Arzneimittel zu schaffen, welches eine gute Wirksamkeit gegen Tumore mit geringer Toxizität verbindet und daher allgemein in der Tumortherapie einsetzbar ist.

Diese Aufgaben werden erfindungsgemäß gelöst durch ein Arzneimittel, welches dadurch gekennzeichnet ist, daß es als Wirkstoff Hexadecylphosphocholin enthält.

Insbesondere für die topische Applikation, aber auch für die Zubereitung als Arzneimittel für andere Applikationsarten hat es sich als besonders günstig herausgestellt, das Hexadecylphosphocholin zusammen mit wenigstens einem Alkylglycerin mit 3 bis 12 Kohlenstoffatomen im Alkylrest, der in Form einer Ethergruppe an eine der primären oder sekundären OH-Gruppen des Glycerins gebunden vorliegen kann, einzusetzen. Derartige Alkylglycerine steigern beziehungsweise verbessern die Wirkung des Hexadecylphosphocholins synergistisch. Bevorzugt werden hierbei Alkylglycerine mit 3 bis 9 C-Atomen allein oder in Mischung verwendet.

Besonders günstige Wirkungen besitzt daher ein synergistisch wirkendes Arzneimittel, welches

a) Hexadecylphosphocholin und

b) ein Alkylglycerin der allgemeinen Formel I

$$\begin{array}{l} H_2C - O - R_1 \\ \quad | \\ \ HC - O - R_2 \\ \quad | \\ H_2C - OH \end{array}$$

in der einer der Reste $R_1$ und $R_2$ eine Alkylgruppe mit 3 bis 12 C-Atomen und der andere Rest ein H-Atom bedeutet, enthält,

sowie gegebenenfalls weitere übliche pharmakologische Zusatz- und Verdünnungsmittel.

Eine derartige Mischung wird im folgenden auch als Kaskade bezeichnet.
Der Gehalt an Hexadecylphosphocholin in mg/ml Kaskade wird durch einen nachgesetzten Index bezeichnet, derart,

...

daß zum Beispiel ein Kaskadengemisch, welches 5 mg/ml Hexadecylphosphocholin enthält, als $Kaskade_5$, ein Gemisch mit 200 mg Verbindung Hexadecylphosphocholin pro ml Kaskade als $Kaskade_{200}$ bezeichnet wird.

Die Herstellung der Alkylglycerine ist bekannt, beispielsweise aus der DE-OS 33 43 530.8. Beispielsweise werden Alkylglycerin-Wasser-Mischungen, welche zum Beispiel Nonylglycerin, Octylglycerin, Hexylglycerin, Pentylglycerin, Propylglycerin und Ethylglycerin enthalten, bevorzugt. Vorzugsweise enthalten solche wäßrigen Mischungen 3 der genannten Glycerinether und zwar einen niederen (Ethyl, Propyl), einen mittleren (Pentyl, Hexyl) und einen höheren (Nonyl, Octyl), wobei die Gewichtsmenge an dem niederen Ether etwa so groß ist wie die Summe der Gewichtsmengen an den beiden anderen Glycerinethern. Die Wassermenge ist etwa gleich der Menge an dem niederen Glycerinether und beträgt beispielsweise die Hälfte der Gesamtmenge an den vorliegenden Glycerinethern. Beispiele für solche Glycerinether-Wasser-Mischungen sind im folgenden angeführt:

| | Wasser | | Glycerin-Propyl-ether | | Glycerin-Hexyl-ether | | Glycerin-Nonyl-ether |
|---|---|---|---|---|---|---|---|
| Gewichts-teile | 2 | : | 2 | : | 1 | : | 1 |

| | Wasser | | Glycerin-Ethyl-ether | | Glycerin-Pentyl-ether | | Glycerin-Octyl-ether |
|---|---|---|---|---|---|---|---|
| Gewichts-teile | 2 | : | 2 | : | 1 | : | 1 |

...

Zur topischen Applikation sind die erfindungsgemäßen Arzneimittel in besonderem Maße geeignet. Um Hauttumoren beziehungsweise Hautmetastasen mit diesem Arzneimittel zu behandeln, werden die betroffenen Hautbezirke beispielsweise mit $Kaskade_5$ bis $Kaskade_{200}$ zwei- bis dreimal täglich eingerieben. Schädliche Nebenwirkungen konnten bisher nicht beobachtet werden, auch nicht bei Patienten, die über einen Zeitraum von 3 Monaten behandelt wurden. Die Remission der Hautmetastasen ist begleitet von einer Normalisierung der Haut, wie durch Gewebeschnitte eindeutig nachgewiesen werden konnte. Mehrere Patientinnen mit Hautmetastasen wurden auf diese Weise behandelt und hierbei ein vollständiges Verschwinden der Mammakarzinom-Hautmetastasen beobachtet.

Die topische Behandlung mit dem erfindungsgemäß bevorzugten Mittel in der Formulierung $Kaskade_5$ bis $Kaskade_{200}$ läßt sich auch für die Behandlung von inneren Tumoren beziehungsweise Metastasen durch großflächiges Einreiben der Haut anwenden. Über die Resorption durch die Haut werden hierbei therapeutisch wirksame Blutspiegel erreicht. Ein Vorteil dieser Applikationsart liegt darin, daß die Zubereitungen $Kaskade_5$ bis $Kaskade_{200}$ von der Haut problemlos toleriert werden.

Diese bevorzugte Zubereitungsart des erfindungsgemäßen Arzneimittels in Form der Lösungen $Kaskade_5$ bis $Kaskade_{200}$ eignet sich auch gut für die Herstellung von Suppositorien für die rektale Einführung. Auch hiermit lassen sich innere Tumore beziehungsweise innere Metastasen gut behandeln.

Eine andere Anwendungsart der erfindungsgemäßen Arzneimittel besteht in der Instillation in präformierte

...

Körperhöhlen. Diese Anwendungsart eignet sich besonders für Pleurakarzinosen, malignen Aszites, maligne Perikardergüsse und Blasenkarzinome. In diesem Fall wird das Hexadecylphosphocholin entweder allein oder in Verbindung mit üblichen Träger- und Verdünnungsmitteln, insbesondere auch mit Kaskade eingesetzt werden.

Zur systematischen Applikation kommt zum Beispiel orale oder intravenöse Verabreichung in Betracht.

Für die orale Verabreichung wird das Hexadecylphosphocholin beispielsweise in Form einer Trinklösung angewendet. Als Träger eignen sich beispielsweise Milch, Kakao, Fruchtsaft oder Trinkwasser. Die Herstellung einer solchen Trinklösung kann zum Beispiel durch Verdünnen einer konzentrierten alkoholischen Lösung von Hexadecylphosphocholin mit Wasser oder einem anderen der zuvor genannten Mittel erfolgen. Bei Ratten führten Tagesdosen von 20, 40 und 60 mg/kg Körpergewicht Hexadecylphosphocholin zu einer vollständigen Remission von chemisch induzierten Mammakarzinomen. Hierbei erweist sich das Hexadecylphosphocholin als besser wirksam und besser verträglich als zum Beispiel 1-Octadecyl-2-methyl-rac-glycero-3-phosphochlolin. Bei dem für diese Versuche verwendeten Tumormodell handelt es sich um ein sogenanntes hartes Modell. Dies bedeutet, daß die an diesem Modell erstellten Befunde auch auf die humane Situation übertragbar sind.

Für die intravenöse Verabreichung über die intravenöse Infusionstherapie wird das Hexadecylphosphocholin zweckmäßig in physiologischer Kochsalzlösung angewendet. Auch andere Infusionslösungen können hierbei angewendet werden. Dosis am Menschen für solche Lösungen ist beispielsweise 1 - 10 mg/kg Körpergewicht.

...

Schließlich können mehrere Applikationsarten des erfindungsgemäßen Arzneimittels kombiniert angewendet werden, wobei die besondere topische Verträglichkeit dazu führt, daß einerseits ein Einreiben der Haut mit einer der anderen Applikationsformen kombiniert angewendet wird.

Eine weitere Trägermischung für das Hexadecylphosphocholin, die sich besonders bewährt hat, besteht aus einer Mischung von etwa 4 Gewichtsteilen Wasser, 4 Gewichtsteilen Propylglycerin und je 2 Gewichtsteilen Hexylglycerin und Nonylglycerin.

Die topische Anwendung des erfindungsgemäßen Arzneimittels in der besonders bevorzugten Zubereitungsform $Kaskade_5$ bis $Kaskade_{200}$ über einen Zeitraum von mehreren Monaten zeigte, daß die lokale Toxizität sich auf ein verstärktes Abschuppen der Haut, ähnlich wie bei der lokalen Anwendung von Acetylsalicylsäure, beschränkt.

Die Erfindung stellt somit ein neues Arzneimittel zur Behandlung von Tumoren zur Verfügung und liefert hierbei nicht nur überhaupt ein weiteres Antitumormittel, sondern bringt erstmals ein auch bei topischer Anwendung im klinischen Versuch nachgewiesenermaßen wirksames Mittel. Hierdurch werden für die Behandlung von Tumorpatienten neue Möglichkeiten eröffnet.

Zur Herstellung von entsprechenden Arzneimitteln wird das Hexadecylphosphocholin mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht. Dies erfolgt zum Beispiel dadurch, daß das Hexadecylphosphocholin zusammen mit üblichen Träger- und/oder Verdünnungs-

...

beziehungsweise Hilfsstoffen bei Temperaturen zwischen 20 und 120° C, vorzugsweise 30 - 100° C vermischt beziehungsweise homogenisiert, die so erhaltene Mischung zur Herstellung von Zubereitungen, die in der Dosierungseinheit 5 bis 2000 mg, vorzugsweise 10 bis 500 mg, insbesondere 30 bis 400 mg Hexadecylphosphocholin enthalten, in Hohlzellen entsprechender Größe ausgießt oder in Kapseln entsprechender Größe abfüllt oder granuliert und dann, gegebenenfalls unter Zusatz von weiteren üblichen Hilfsstoffen zu Tabletten verpreßt.

Zum Beispiel dadurch, daß man Hexadecylphosphocholin mit einem oder mehreren der folgenden Stoffe: Stärke, Cellulose, Lactose, Formalin-Casein, modifizierte Stärke, Magnesiumstearat, Calciumhydrogen phosphat, hochdisperse Kieselsäure, Talkum, Phenoxyethanol vermischt, die erhaltene Mischung, gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylsorbitanmonooleat enthält, granuliert, das Granulat gegebenenfalls mit einem oder mehreren der obengenannten Hilfsstoffe homogenisiert, und diese Mischung zu Tabletten verpresst oder in Kapseln abfüllt, wobei solche Tabletten oder Kapseln in der Dosierungseinheit jeweils 5 bis 2000 mg Hexadecylphosphocholin enthalten, oder daß man Hexadecylphosphocholin nach Zusatz von Sojalecithin sowie gegebenenfalls 0,1 - 0,5 Gewichtsteilen Phenoxyethanol (bezogen auf einen Gewichtsteil Hexadecylphosphocholin) bei Temperaturen zwischen 33 - 37° C in geschmolzenem Hartfett suspendiert und homogenisiert und anschließend die Mischung in Hohlzellen ausgießt, wobei die Dosierungseinheit 5 bis 2000 mg Hexadecylphosphocholin sowie gegebenenfalls 0,1 - 0,5 Gewichtsteile Phenoxyethanol (bezogen auf einen Gewichtsteil Hexadecylphosphocholin) enthält, oder

...

daß man Hexadecylphosphocholin bei einer Temperatur zwischen 50 bis 120° C, vorzugsweise 50 bis 100° C, gegebenenfalls in Gegenwart eines oder mehrerer Emulgatoren und/oder 0,1 - 0,5 Gewichtsteilen Phenoxyethanol (bezogen auf einen Gewichtsteil Hexadecylphosphocholin mit mindestens einem der folgenden Stoffe homogenisiert: Paraffin, Vaseline, aliphatischer Alkohol mit 12 bis 25 C-Atomen, aliphatische Monocarbonsäure mit 15 bis 20 C-Atomen, Sorbitanmonopalmitat, Polyoxyethylenpolyolfettsäureester, und die erhaltene Mischung zwischen 50 und 120° C mit Wasser, gegebenenfalls unter Zusatz eines mehrwertigen niederen aliphatischen Alkohols und/oder Phenoxyethanol emulgiert; oder daß man Hexadecylphosphocholin in Wasser oder Pflanzenöl, gegebenenfalls in Gegenwart von 0,1 - 0,5 Gewichtsteilen Phenoxyethanol (bezogen auf einen Gewichtsteil Hexadecylphosphocholin) sowie gegebenenfalls in Gegenwart eines Emulgators, bei Temperaturen zwischen 30 - 100° C auflöst, und gegebenenfalls die so erhaltene Lösung mit soviel Wasser oder Pflanzenöl auffüllt, daß die Endlösung 0,05 bis 10 Gewichtsprozent, vorzugsweise 0,1 bis 5 Gewichtsprozent Hexadecylphosphocholin enthält.

Als Emulgatoren kommen zum Beispiel in Frage: nichtionogene Emulgatoren sowie ionogene Emulgatoren. Bei den nichtionogenen Emulgatoren handelt es sich beispielsweise um Triglyceridgemische von gesättigten Pflanzenfettsäuren mit $C_8$, $C_{10}$ und $C_{12}$ oder um Emulgatoren auf der Basis von Polyadditionsprodukten des Ethylenoxids, wie zum Beispiel alkyl- und acylsubstituierte Polyadditionsprodukte des Ethylenoxids, Polyethylenglykol-fettsäureester, Umsetzungsprodukte von Ethylenoxid mit Ricinusöl beziehungsweise hydriertem Ricinusöl, Ester von hydrierten Ricinusöl-

...

fettsäuren mit oxyethyliertem Glycerin. Weiterhin kann os sich um Emulgatoren auf Basis von Fettsäureamiden oder Fettsäurekondensationsprodukten mit hydrophilen Gruppen handeln. Als ionogene Emulgatoren kommen zum Beispiel Emulgatoren auf Basis von Fettsäuremonoestern des Glycerins oder anderer mehrwertiger Alkohole (Lunacera alba) in Frage.

Falls bei der wie oben angegebenen Herstellung der Arzneimittel das Hexadecylphosphocholin in Gegenwart von einem Glycerinether der Formel I oder einer Mischung von solchen Glycerinethern der Formel I verwendet werden, wird eine synergistische Wirkungssteigerung der Antitumorwirkung beobachtet.
Hierzu wird das Hexadecylphosphocholin mit 1 bis 30, vorzugsweise 2 bis 20 Gewichtsteilen (bezogen jeweils auf einen Gewichtsteil Hexadecylphosphocholin) von mindestens einem Glycerinether der Formel I oder einem Gemisch solcher Glycerinether sowie gegebenenfalls 0,5 - 30, vorzugsweise 1 - 20 Gewichtsteilen Wasser (ebenfalls bezogen auf einen Gewichtsteil Hexadecylphosphocholin) verwendet. Diese Vermischung mit den Glycerinethern kann bei der Herstellung der entsprechenden Arzneimittel am Anfang erfolgen, aber gegebenenfalls auch in einem späteren Herstellungsstadium.

...

Das Hexadecylphosphocholin zeigt beispielsweise eine gute Wirkung am 7,12-Dimethylbenzanthracen induzierten Brustdrüsenkrebs der Ratte; ebenso am Methyl-nitrosoharnstoff-induzierten Mammacarcinom der Ratte.

Beispielsweise wird bei obengenannter Versuchsmethode bei einer Dosis von 10 mg/kg Körpergewicht Ratte ein Wachstumsstillstand der Tumore, bei höheren Dosen auch ein völliges Verschwinden der Geschwülste erzielt.

Die niedrigste, bereits wirksame Dosis in dem obenangegebenen Tierversuch ist beispielsweise

5 mg/kg oral
5 mg/kg intravenös.

Als allgemeiner Dosisbereich für die Wirkung (Tierversuch wie oben) kommt beispielsweise in Frage:

5 - 50 mg/kg oral, insbesondere 15 - 32 mg/kg
5 - 50 mg/kg intravenös, insbesondere 15 - 32 mg/kg.

Die Wirkungsrichtung der erfindungsgemäßen Verbindungen ist mit der Wirkung des bekannten Arzneimittelwirkstoffs TAMOXIFEN vergleichbar, jedoch bestehen hierzu insbesondere folgende Unterschiede: Die Wirkung ist stärker und von längerer Dauer als die von TAMOXIFEN.

Indikationen, für die die erfindungsgemäßen Verbindungen in Betracht kommen können: Brustdrüsenkrebs und andere menschliche Krebsarten.

...

Die pharmazeutischen Zubereitungen enthalten im allgemeinen zwischen 5 - 2000 mg, beispielsweise 10 - 400 mg Hexadecylphosphocholin.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 40 und 400 mg oder Lösungen, die zwischen 0,1 % bis 5 % an aktiver Substanz enthalten.

Die Einzeldosis an Hexadecylphosphocholin kann beispielsweise liegen

a) bei oralen Arzneiformen zwischen 5 - 100 mg/kg Körpergewicht, vorzugsweise 15 - 50 mg/kg Körpergewicht,

b) bei parenteralen Arzneiformen (zum Beispiel intravenös, intramuskulär, zwischen 5 - 100 mg/kg Körpergewicht,

c) bei Arzneiformen zur lokalen Applikation auf die Haut und Schleimhäute (zum Beispiel in Form von Lösungen, Lotionen, Emulsionen, Salben und so weiter) zwischen 50 - 2000 mg, vorzugsweise 80 - 1500 mg.

...

Beispielsweise können 3 mal täglich 1 Tablette mit einem Gehalt von 40 - 400 mg wirksamer Substanz oder zum Beispiel bei intravenöser Injektion 1 - 5 mal täglich eine Ampulle von 1 - 5 ml Inhalt mit 50 - 250 mg Substanz empfohlen werden. Bei oraler Verabreichung ist die minimale tägliche Dosis beispielsweise 120 mg; die maximale tägliche Dosis bei oraler Verabreichung soll nicht über 100 mg/kg Körpergewicht liegen.

Die akute Toxizität des Hexadecylphosphocholins an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 200 und 450 mg/kg Körpergewicht.

Die Arzneimittel können in der Humanmedizin, der Veterinärmedizin sowie in der Landwirtschaft allein oder im Gemisch mit anderen pharmakologisch aktiven Stoffen verwendet werden.

Die Erfindung wird durch die folgenden Beispiele erläutert.

B e i s p i e l 1

Herstellung von Hexadecylphosphocholin·$H_2O$

a) Hexadecylphosphoethanolamin
(Phosphorylierung, Ringschluß und Ringöffnung)

Hexadecanol (1 Mol, 243 g) und Triethylamin (1,8 Mol, 180 g) werden in 1,5 l THF (Tetrahydrofuran) gelöst und tropfenweise zu einer stark gerührten Lösung von Phosphoroxychlorid (1,2 Mol, 184 g) in 120 ml THF so zugegeben, daß die Temperatur im Reaktionsgefäß (Dreihals, 5 l, mit Tropftrichter, Thermometer und Rührer) 10°C nicht übersteigt. Zur Beschleunigung des Vorgangs wird das Reaktionsgefäß mit einer Eis-Kochsalzmischung gekühlt. Unmittelbar nach dem Eintropfen ist die Reaktion abgeschlossen (Nachweis über DSC in Ether: Rf-Werte von 0,8 für das Ausgangsprodukt, von 0,0 für das Reaktionsprodukt nach Hydrolyse mit Wasser).

Man entfernt das Eisbad und tropft in das Reaktionsgemisch unter starkem Rühren eine Lösung von Ethanolamin (1,5 Mol, 92 g) und Triethylamin (1,8 Mol, 180 g) in 1 l Dioxan so ein, daß die Temperatur im Reaktionsgefäß auf 65 bis 70°C steigt. Dann ist die Ringbildung abgeschlossen (Nachweis durch DSC in Ether: Rf-Wert von 0,2). Man filtriert von ausgefallenem Triethylaminhydrochlorid noch warm ab und versetzt das Filtrat bei 40 bis 50°C mit 1,5 l 2N Ameisensäure. Nach 15 Minuten ist die Ringöffnung abgeschlossen (Nachweis durch DSC in Ether: Rf-Wert 0,0; DSC in Chloroform/Methanol/Essigsäure/Wasser 100:60:20:5 per Vol.:Rf-Wert 0,8). Man kühlt auf -20°C und filtriert vom Niederschlag ab, der aus weitgehend reinem Hexadecylphosphoethanolamin besteht. Bei leichten Verunreinigungen wird eine chromatographische Reinigung angeschlossen (siehe Beispiel 2). Mikroanalyse (MG 365,50):

| | | | | |
|---|---|---|---|---|
| ber. (%): | C 59,15 | H 11,03 | N 3,83 | P 8,48 |
| gef. (%): | 59,01 | 10,95 | 3,79 | 8,31 |

b) (Methylierung von 1)

Die nach Beispiel 1 erhaltenen Kristalle werden ohne weitere Reinigung in 1,2 l 2-Propanol und 0,4 l Dichlormethan aufgenommen. Man versetzt die Suspension der Kristalle unter starkem Rühren mit Kaliumkarbonat (4 Mol, 560 g) in 1 l Wasser. Das zweiphasige Reaktionsgemisch wird mit Dimethylsulfat (4 Mol, 500 g) tropfenweise und unter Rühren so versetzt, daß die Temperatur 40°C nicht übersteigt. Die Reaktion ist 60 Minuten nach dem Eintropfen beendet (Nachweis durch DSC in Chloroform/Methanol/25%igem Ammoniak 50:50:5 per Vol.: Rf-Wert 0,3). Nach Phasenseparation bei 20°C enthält die

obere Phase das Produkt. Man entfernt das Lösungsmittel am Rotationsverdampfer unter Vakuum und chromatographiert den viskosen Rückstand an Kieselgel (Merck Art. 7733, Kieselgel 60, Korngröße 0,2 bis 0,5 mm).

Chromatographie

Kieselgel, 2 kg, werden mit Chloroform/Methanol/25%igem Ammoniak (200/15/1 per Vol.) versetzt und in eine Chromatographiesäule gefüllt. Man löst das viskose Öl in 800 ml des obigen Lösungsmittelgemisches und gibt das Rohprodukt auf die Säule (unlösliche Anteile werden vorher abfiltriert). Man eluiert mit Fließmitteln steigender Polarität bis die Verunreinigungen ausgewaschen sind. Das Produkt wird schließlich mit Chloroform/Methanol/25%igem Ammoniak (50/50/5 per Vol.) eluiert. Die vereinigten Eluate werden einrotiert und mit Toluol das restliche Wasser entfernt. Der Rückstand wird in 600 ml Dichlormethan aufgenommen und mit 4 l Aceton versetzt. Die bei -20°C abgeschiedenen Kristalle werden mit kaltem Aceton gewaschen, dann mit Pentan und im Vakuum getrocknet. Die Ausbeute an reinem Hexadecylphosphocholin beträgt 250 g (ca. 70 % bezogen auf Hexadecylglycerin).

Mikroanalyse (MG 407,58):

| | | | | |
|---|---|---|---|---|
| ber. (%): | C 59,27 | H 11,37 | N 3,29 | P 7,28 |
| gef. (%): | 58,98 | 11,31 | 3,21 | 7,11 |

Beispiele für pharmazeutische Zubereitungen

Beispiel für eine Lösung:
25 g 1-n-Propyloxy-2,3-Propandiol, 12,5 g 1-n-Hexyloxy-2,3-propandiol, 12,5 g 1-n-Nonyloxy-2,3-propandiol, 44 g Wasser und 1 g Phenoxyethanol werden gemischt und 5 g Hexadecylphosphocholin in dieser Mischung gelöst. Die Lösung wird durch Filtration über geeignete Filter von sichtbaren Partikeln befreit.
1 g Lösung enthält 50 mg Hexadecylphosphocholin.

Beispiel für eine Salbe:
5 g Substanz Hexadecylphosphocholin werden in 35 g dickflüssigem Paraffin suspendiert, 30 g emulgierender Cetylstearylalkohol und 30 g weißes Vaselin werden zugesetzt und geschmolzen. Diese Schmelze wird bis zum Erkalten gerührt. Eine homogene Wirkstoffverteilung wird durch Bearbeitung der erkalteten Schmelze mittels eines geeigneten Homogenisiergerätes (zum Beispiel Dreiwalzenstuhl) erreicht.
1 g der hydrophilen Salze enthält 50 mg Hexadecylphosphocholin.

Beispiel für eine Emulsion:
11,83 g 1-n-Propyloxy-2,3-propandiol, 5,91 g 1-n-Hexyloxy-2,3-propandiol, 5,91 g 1-n-Nonyloxy-2,3-propandiol, 20,35 g Wasser und 1,0 g Phenoxyethanol werden gemischt und 5 g Hexadecylphosphocholin in dieser Mischung gelöst. Auf einem Wasserbad werden 30 g weißes Vaselin, 15 g Cetylalkohol und 5 g Sorbitanmonopalmitat geschmolzen, auf 70° C erwärmt und die ebenfalls auf 70° C erwärmte Wirkstofflösung mit Hilfe eines hochtourigen Dispergiergerätes in der Fettphase emulgiert. Anschließend wird unter Rühren die Creme auf 30° C abgekühlt.
1 g Wasser in Öl-Creme enthält 50 mg Hexadecylphosphocholin.

Beispiel für Kapseln:

1,25 kg Hexadecylphosphocholin werden in 5 kg Chloroform gelöst und in dieser Lösung 1,25 kg Aerosil suspendiert. Anschließend wird das Lösungsmittel im Vakuum abgezogen. Die trockene Masse wird durch ein 1 mm-Sieb gegeben und noch einmal im Vakuum bei 30° C getrocknet, um letzte Lösungsmittelrückstände zu entfernen. Dieses Granulat wird in bekannter Weise auf einer geeigneten Kapselmaschine in Gelatine Hartkapseln der Größe 00 zu 500 mg abgefüllt.

Eine Kapsel enthält 250 mg Hexadecylphosphocholin.

Beispiel für ein Lyophilisat:

In 3 Liter Wasser für Injektionszwecke werden unter Stickstoffbegasung 500 g Mannit gelöst, 50 g Hexadecylphosphocholin mit Hilfe eines hochtourigen Homogenisiergerätes dispergiert und mit Wasser für Injektionszwecke auf 4 Liter aufgefüllt. Diese milchige Dispersion wird durch Ultraschallbehandlung oder mit Hilfe eines Spalthomogenisators in ein leicht opaleszierendes kolloiddisperses System überführt.

Unter aseptischen Bedingungen wird nun über ein Membranfilter von 0,22 µm Porenweite sterilfiltriert und zu 40 ml in 100 ml-Injektionsflaschen unter Stickstoffbegasung abgefüllt. Die Flaschen versieht man mit Gefriertrocknungsstopfen und lyophilisiert in einer geeigneten Anlage. Nach der Trocknung wird mit sterilem, getrockneten Stickstoff begast und die Flaschen in der Anlage verschlossen. Die Stopfen werden mit einer Bördelkappe gesichert.

Für die intravenöse Anwendung wird das Lyophilisat in 100 ml Wasser für Injektionszwecke rekonstituiert.

1 Flasche enthält 500 mg Hexadecylphosphocholin.

Arzneimittel mit Antitumorwirkung

BEZEICHNUNG GEÄNDERT
siehe Titelseite

Patentansprüche:

1. Antitumorwirksames Arzneimittel, enthaltend Hexadecylphosphocholin als Wirkstoff sowie gegebenenfalls weitere übliche pharmazeutische Zusatz-, Träger- und/oder Verdünnungsstoffe.

2. Arzneimittel, dadurch gekennzeichnet,

a) daß es als Wirkstoff Hexadecylphosphocholin enthält und

b) ein Alkylglycerin der allgemeinen Formel I

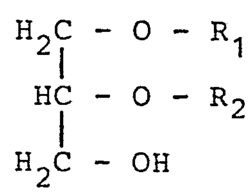

in der einer der Reste $R_1$ und $R_2$ eine Alkylgruppe mit 3 bis 12 C-Atomen und der andere Rest ein H-Atom bedeutet, enthält,

sowie gegebenenfalls weitere übliche pharmazeutische Zusatz-, Träger- und/oder Verdünnungstoffe.

...

3. Arzneimittel nach einem oder mehreren der vorangegangenen Ansprüche, dadurch gekennzeichnet, daß es ein Alkylglycerin-Gemisch enthält aus Nonyl- beziehungsweise Octylglycerin, Hexyl- beziehungsweise Pentylglycerin und Propyl- beziehungsweise Ethylglycerin sowie Wasser.

4. Arzneimittel nach einem oder mehreren der vorangegangenen Ansprüche, dadurch gekennzeichnet, daß es in der Dosierungseinheit 5 - 2000 mg, insbesondere 10 - 500 mg Hexadecylphosphocholin enthält.

5. Arzneimittel nach einem oder mehreren der vorangegangenen Ansprüche, dadurch gekennzeichnet, daß es zur topischen Behandlung von Hauttumoren 5 bis 200 mg Hexadecylphosphocholin pro ml Alkylglycerin der Formel I beziehungsweise eines entsprechenden Alkylglyceringemisches enthält.

6. Arzneimittel nach einem oder mehreren der vorangegangenen Ansprüche, dadurch gekennzeichnet, daß es zur oralen Behandlung von Tumoren als Trinklösung formuliert wird mit einer Tagesdosis zwischen 5 und 100 mg/kg Körpergewicht.

...

7. Arzneimittel nach einem oder mehreren der vorangegangenen Ansprüche, dadurch gekennzeichnet, daß es zur intravenösen Behandlung von Tumoren Hexadecylphosphocholin in einer Menge von 5 - 100 mg/kg Körpergewicht in physiologischer Kochsalzlösung enthält.

8. Verfahren zur Herstellung eines (antitumorwirksamen) Arzneimittels, dadurch gekennzeichnet, daß man Hexadecylphosphocholin mit üblichen physiologisch verträglichen Träger- und/oder Verdünnungs- beziehungsweise Hilfsstoffen bei Temperaturen zwischen 20 und 120° C vermischt beziehungsweise homogenisiert und gegebenenfalls die so erhaltene Mischung zur Herstellung von Zubereitungen, die in der Dosierungseinheit 5 bis 2000 mg Hexadecylphosphocholin enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

9. Verfahren zur Herstellung eines (antitumorwirksamen) Arzneimittels, dadurch gekennzeichnet, daß man Hexadecylphosphocholin mit einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum, Phenoxyethanol

...

vermischt, die erhaltene Mischung, gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitanmonooleat enthält, granuliert, das Granulat gegebenenfalls mit einem oder mehreren der obengenannten Hilfsstoffe homogenisiert, und diese Mischung zu Tabletten verpresst oder in Kapseln abfüllt, wobei die Tabletten oder Kapseln in der Dosierungseinheit jeweils 5 bis 2000 mg Hexadecylphosphocholin enthalten.

10. Verfahren zur Herstellung eines (antitumorwirksamen) Arzneimittels,
dadurch gekennzeichnet,
daß man Hexadecylphosphocholin mit Sojalecithin sowie gegebenenfalls 0,1 - 0,5 Gewichtsteilen Phenoxyethanol (bezogen auf einen Gewichtsteil Hexadecylphosphocholin) bei Temperaturen zwischen 33 - 37° C in geschmolzenem Hartfett suspendiert und homogenisiert und anschließend die Mischung in Hohlzellen ausgießt, wobei die Dosierungseinheit 5 bis 2000 mg Hexadecylphosphocholin enthält.

11. Verfahren zur Herstellung eines (antitumorwirksamen) Arzneimittels (Salze, Creme, Emulsion),
dadurch gekennzeichnet,
daß man Hexadecylphosphocholin bei einer Temperatur zwischen 50 bis 120° C, gegebenenfalls in Gegenwart eines oder mehrerer Emulgatoren und/oder 0,1 - 0,5 Gewichtsteilen Phenoxyethanol (bezogen auf 1 Gewichtsteil Hexadecylphosphocholin) mit mindestens

...

einem der folgenden Stoffe homogenisiert: Paraffin, Vaseline, aliphatischer Alkohol mit 12 bis 25 C-Atomen, Sorbitanmonopalmitat, aliphatische Monocarbonsäure mit 15 bis 20 C-Atomen, Polyoxyethylenpolyolfettsäureester, und gegebenenfalls unter Zusatz eines mehrwertigen niederen aliphatischen Alkohols emulgiert.

12. Verfahren zur Herstellung einer (antitumorwirksamen) Lösung,
dadurch gekennzeichnet,
daß man Hexadecylphosphocholin gegebenenfalls in Gegenwart von 0,1 - 0,5 Gewichtsteilen Phenoxyethanol (bezogen auf einen Gewichtsteil Hexadecylphosphocholin) sowie gegebenenfalls in Anwesenheit eines Emulgators, bei Temperaturen zwischen 30 - 100° C auflöst, und gegebenenfalls die so erhaltene Lösung mit soviel Wasser oder Pflanzenöl auffüllt, daß die Endlösung 0,1 - 5 Gewichtsprozent an Hexadecylphosphocholin enthält.

13. Verfahren zur Herstellung eines Arzneimittels gemäß einem oder mehreren der vorangegangenen Verfahrensansprüche,
dadurch gekennzeichnet,
daß man bei der Herstellung zusätzlich noch ein Alkylglycerin der Formel I

$$\begin{array}{l} H_2C - O - R_1 \\ \;\;| \\ \;HC - O - R_2 \\ \;\;| \\ H_2C - OH \end{array}$$

...

in der einer der Reste $R_1$ und $R_2$ eine Alkylgruppe mit 3 bis 12 C-Atomen und der andere Rest ein H-Atom bedeutet, oder ein Gemisch solcher Alkylglycerine sowie gegebenenfalls Wasser verwendet, wobei man 1 - 30 Gewichtsteile Alkylglycerin der Formel I beziehungsweise eines entsprechenden Alkylglyceringemisches und gegebenenfalls 1 - 30 Gewichtsteile Wasser, jeweils bezogen auf einen Gewichtsteil Hexadecylphosphocholin, verwendet.

...